(19) Europäisches Patentamt / European Patent Office / Office européen des brevets

(11) **EP 4 003 438 B1**

(12) **EUROPEAN PATENT SPECIFICATION**

(45) Date of publication and mention
of the grant of the patent:
**23.11.2022 Bulletin 2022/47**

(21) Application number: **20747344.8**

(22) Date of filing: **10.07.2020**

(51) International Patent Classification (IPC):
**A61L 27/22** (2006.01)    **A61L 27/26** (2006.01)
**A61L 27/36** (2006.01)    **A61L 27/50** (2006.01)
**B33Y 70/00** (2020.01)    **B33Y 80/00** (2015.01)
**C12N 5/00** (2006.01)

(52) Cooperative Patent Classification (CPC):
(C-Sets available)
**B33Y 70/00; A61L 27/222; A61L 27/26;**
**A61L 27/3633; A61L 27/50; B33Y 80/00;**
**C12N 5/0068;** A61L 2300/442; C12N 2529/10;
C12N 2535/10                                    (Cont.)

(86) International application number:
**PCT/EP2020/069600**

(87) International publication number:
**WO 2021/018553 (04.02.2021 Gazette 2021/05)**

(54) **PHOTOCURABLE COMPOSITION**

LICHTHÄRTBARE ZUSAMMENSETZUNGEN

COMPOSITION PHOTODURCISSABLE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **31.07.2019 EP 19189493**

(43) Date of publication of application:
**01.06.2022 Bulletin 2022/22**

(73) Proprietor: **Freie Universität Berlin**
**14195 Berlin (DE)**

(72) Inventors:
• **WEINHART, Marie**
  **12435 Berlin (DE)**
• **ELOMAA, Laura**
  **12049 Berlin (DE)**

(74) Representative: **Maikowski & Ninnemann**
**Patentanwälte Partnerschaft mbB**
**Postfach 15 09 20**
**10671 Berlin (DE)**

(56) References cited:
**US-A1- 2019 119 429**

• **KLOTZ BARBARA J ET AL:**
**"Gelatin-Methacryloyl Hydrogels: Towards**
**Biofabrication-Based Tissue Repair", TRENDS IN**
**BIOTECHNOLOGY, ELSEVIER PUBLICATIONS,**
**CAMBRIDGE, GB, vol. 34, no. 5, 9 February 2016**
**(2016-02-09), pages 394-407, XP029516979, ISSN:**
**0167-7799, DOI: 10.1016/J.TIBTECH.2016.01.002**
• **DATABASE WPI Week 201961 Thomson**
**Scientific, London, GB; AN 2019-57370R**
**XP002797182, & CN 109 908 086 A (UNIV**
**HUAQIAO) 21 June 2019 (2019-06-21)**

Processed by Luminess, 75001 PARIS (FR)

**(Cont. next page)**

(52) Cooperative Patent Classification (CPC): (Cont.)

C-Sets
**A61L 27/26, C08L 67/04;**
**A61L 27/26, C08L 89/06**

**Description**

The invention relates to photocurable composition for 3D printing according to the preamble of claim 1, to the use of such a photocurable composition according to the preamble of claim 11, and to a method for manufacturing a three-dimensional object from such a composition according to the preamble of claim 13.

Stereolithography (SLA)-based 3D printing enables precise transfer of CAD models into a 3D structure and is therefore a great help in fabrication of tissue engineering scaffolds. In digital light processing (DLP) SLA, a high-definition projector allows fast, high-resolution curing of a whole layer at once, and therefore the printing resolution is not limited by laser- or extruder-related factors, such as a size or a speed of a printing head. However, to obtain a high resolution, the material choice is critical: the resin polymer has to have a high density of photocrosslinkable groups, the polymer concentration has to be high enough to ensure a close proximity of the reacting groups, and the viscosity of the resin has to be low enough to allow its free flow.

Low molecular weight, star-shaped poly($\varepsilon$-caprolactone) methacrylate (PCL-MA) is a great material for SLA-based 3D printing as it can be printed without solvents into high-resolution 3D structures. However, due to its high hydrophobicity, it is not water-permeable, and because of lack of cell adhesive peptides, it often requires surface modification to improve cell adhesion properties.

Gelatin methacryloyl (GelMA), on the other hand, is a chemically modified biopolymer derived from collagen that is the most abundant extra-cellular matrix (ECM) protein and inherently contains integrin-binding ligands and matrix metalloproteinase-responsive peptides. Physically crosslinked GelMA hydrogels can be stabilized by covalent, free radical-initiated photocrosslinking of methacryloyl groups, which prevents their disintegration at body temperature. However, in an aqueous solution, GelMA has to be dissolved at a low polymer concentration to prevent its temperature-dependent non-covalent gelation and thereby to allow its free flow for SLA 3D printing.

As a crosslinking density of a photocrosslinkable resin decreases with the polymer concentration, SLA 3D printing of low concentrated aqueous GelMA solutions leads to loosely crosslinked networks and thereby an impaired resolution of the resulting 3D scaffolds. US 2019/119429 describes a composition comprising gelatin methacrylate, a silanized biologically active additive, a photoinitiator and a solvent, preferably water, phosphate buffered saline or cell culture media. The composition is combined with cells. This mixture is formed into a three-dimensional scaffold for cell culture or tissue regeneration by 3D printing.

It is an object of the present invention to provide a resin (or ink) for stereolithography that yields a resolution high enough for fabricating cell or tissue scaffolds with fine 3D features and at the same time is permeable enough to allow molecular transport through the material.

This object is achieved with a photocurable composition for 3D printing having the features according to claim 1. Such a composition comprises

- at least one photocurable prepolymer chosen from the group consisting of a) functionalized gelatin bearing a first functional group and b) functionalized decellularized extracellular matrix bearing a second functional group,
- at least one solvent, the solvent being formamide, and
- at least one photoinitiator.

A particular appropriate photoinitiator is a radical forming photoinitiator such as phenylphosphinate, hydroxy ketone, or camphorquinone. Likewise, a cationic photoinitiator, such as an onium salt or an iodonium salt is a particular appropriate photoinitiator. Furthermore, photoacid generators and/or photoacids are appropriate photoinitiators. Suited examples are triphenylsulfonium triflate and pyranine (8-hydroxy-1,3,6-pyrenetrisulfonate, HPTS).

In prior art, typically aqueous solvents are used to solubilize functionalized gelatin or functionalized decellularized extracellular matrix. Unexpectedly and surprisingly, the inventors were able to show that the organic solvent formamide has superior properties with respect to solubilizing functionalized gelatin and/or functionalized decellularized extracellular matrix. Formamide is a highly polar, yet non-reactive solvent having a sufficiently low hydrophilicity so as to be able to also solubilize hydrophobic polymers or prepolymers. It has a high boiling point (around 210 °C) to avoid undesired evaporation during a 3D printing process. Volatile solvents that evaporate to a significant part during 3D printing distort a 3D printing process since upon evaporation of the solvent, the concentration of the prepolymers to be printed significantly varies. This makes the overall 3D printing process unreliable and error-prone.

The inventors were able to show that other highly polar, non-reactive, organic solvents such as dimethlyformamide (DMF), dimethylsulfoxide (DMSO), N-methlypyrrolidone, methanol, acetonitrile, ethylene glycol did not dissolve functionalized gelatin or functionalized decellularized extracellular matrix nor allowed homogenous blending of either prepolymer with pure functionalized poly ($\varepsilon$-caprolactone) (PCL). Therefore, these solvents are not appropriate to carry out the presently claimed invention.

On the other hand, it is apparent for a person skilled in the art that also other organic solvents having a comparable hydrophilicity like formamide and having a comparably high boiling point like formamide are suited to replace formamide. Thus, a person skilled in the art might consider replacing formamide by any solvent having a comparable polarity index (above 4) (i.e., a comparable hydrophilicity) and a comparable boiling point (above 150 °C), in particular by an ionic liquid fulfilling these criteria. Particularly appropriate ionic liquids are those with surfactant properties due to derivatization with long hydrophobic alkyl chains such as tetraalkylammonium based salts with the alkyl group being a butyl or higher alkyl residue, in particular a $C_4$-$C_{20}$ alkyl residue, in particular a $C_5$-$C_{18}$ alkyl residue, in particular a $C_6$-$C_{16}$ alkyl residue, in particular a $C_7$-$C_{14}$ alkyl residue, in particular a $C_8$-$C_{12}$ alkyl residue.

Decellularized extracellular matrix (dECM) is typically a mixture of glycosamino glycans such as hyaluronic acid, proteins such as collagen and proteoglycans such as aggrecan. It is apparent for a person skilled in the art that using any of these dECM ingredients either alone or in any arbitrary combination instead of dECM will also result in the technical effects of the presently described invention.

In an embodiment, the composition further comprises functionalized poly($\varepsilon$-caprolactone) (PCL) bearing a third functional group. PCL can be blended in virtually any desired amount with the functionalized prepolymer. It significantly stabilizes a structure prepared from the composition. Since very fine structures can be printed with functionalized PCL, the overall resolution is increased if PCL is mixed with the functionalized prepolymer.

In an embodiment, the first functional group, the second functional group, and (if functionalized PCL is present in the composition) the third functional group are independently chosen from each other from the group consisting of methacryloyl, methacrylate, methacrylamide, acryloyl (in particular acrylate and acrylamide), thiol, vinyl, vinylene, allyl, alkynyl, epoxide, hydroxyl (alcohol) and conjugated dienyl groups. These functional groups are particularly appropriate to carry out a polymerization or addition reaction so as to serve for crosslinking individual prepolymer molecules with each other. A thiol group can be particularly well combined with a group bearing a double bond or a triple bond between two carbon atoms. An epoxide group can particularly well form a bond with a hydroxyl group so that, in an embodiment, the functional groups are chosen such that at least one functional group is an epoxide and at least one functional group is a hydroxyl. Epoxide and hydroxyl functional residues can be particularly well combined with a photoacid or a photoacid generator. A diene group is, in an embodiment, combined with a dienophile group (typically bearing a double bond).

The first functional group, the second functional group, and/or the third functional group might be the same or a different chemical group.

In an embodiment, the first functional group, the second functional group, and (if functionalized PCL is present in the composition) the third functional group can only be a thiol group if any compound of the composition comprises a functional group chosen from the group consisting of methacryloyl, methacrylate, methacrylamide, acryloyl, vinyl, vinylene, allyl, alkynyl, epoxide, hydroxyl, and conjugated dienyl groups. Thus, in this embodiment at least one functional group is different from a thiol group. While two thiol groups can generally serve for polymerization by forming a disulfide bond, a polymerization in which also a functional group bearing a double bond or a triple bond is involved, is easier formed so that an overall higher stability of the crosslinked polymers can be achieved.

In an embodiment, the photocurable prepolymer is or comprises gelatin methacryloyl (GelMA). GelMA is a widely used and accepted functionalized gelatin that can be easily polymerized due to its methacryloyl functional group(s).

In an embodiment, the functionalized PCL is PCL methacrylate (PCL-MA) which can likewise be easily polymerized due to its methacrylate functional group(s) and might comprise linear, branched, or star-shaped architecture. A mixture of GelMA and PCL-MA is particularly appropriate for carrying out the presently claimed invention in an embodiment.

In prior art, the mechanical stability of GelMA hydrogels has been reinforced with the help of PCL fibers in multi-material tissue scaffolds and GelMA/PCL blends have been used in electrospinning. However, there are no studies so far reporting use of GelMA/PCL hybrid resins in SLA printing, presumably because of scarcity of suitable solvents. As the typical solvents used in electrospinning of GelMA/PCL blend, i.e. hexafluoro-2-propanol (HFIP) and trifluoroethanol, are highly volatile and HFIP is also corrosive, they are not suitable for SLA printing where the constant viscosity of the resin is critical. The present invention relates in an embodiment to a non-volatile hybrid resin of GelMA and PCL-MA in formamide (boiling point 210 °C). The inventors were able to demonstrate the use of this resin in SLA by printing acellular tissue scaffolds that mimic a physiological intestinal villi structure. Swelling, degradation, and permeability of the new material as well as Caco-2 cell adhesion and differentiation were characterized (see exemplary embodiments for details). Finally, as decellularized ECM (dECM) constitutes the natural, better biomimicking cell environment compared to gelatin, the gained knowledge was transferred by the inventors to dECM, and methacryl-functionalized dECM (dECM-MA) was prepared to demonstrate its use as a photocrosslinkable and PCL-MA-blending resin in SLA 3D printing.

In an embodiment, the gelatin methacryloyl is fish gelatin methacryloyl or porcine gelatin methacryloyl. Fish GelMA cannot transmit mammalian diseases; it might therefore be desired to work with fish GelMA. However, the 3D structures so far manufactured with fish GelMA have not yet had the same quality as the structures manufactured on the basis of porcine GelMA, while still being appropriate to carry out the instantly claimed invention. The structures obtained on the basis of the generally more widely used porcine GelMA were somewhat finer and more stable than the structures made from fish GelMA. Porcine GelMA is known for its good cell adhesion properties.

In an embodiment, the composition further comprises a light-absorbing colorant, such as Orasol Yellow 2RLN, Orasol Orange G, or Orange Red C food color. Such a colorant facilitates the control of the 3D printing process since it can be better observed which sections or regions of the composition used as 3D printing resin have already been irradiated by light (in particular if the colorant is photo-bleachable) so that it is easily conceivable for a user in which regions a photopolymerization reaction has been or is initiated. Furthermore, the colorant will absorb part of the irradiated light so that the overall penetration depth of the light is decreased. Thus, the colorant can help controlling the polymerization process in the z direction

In an embodiment, the composition further comprises a crosslinker, such as trimethylpropane trimethacrylate, pentaerythritol tetramethacrylate, or trimethylolpropane tris(3-mercaptopropionate). Such a crosslinker enables the formation of additional bonds between the individual prepolymer molecules. This leads to an increased stability of the formed polymer and to a higher stability of any structures printed from the prepolymer and finally formed by the crosslinked polymer.

In order to achieve a particularly simple polymerization of the optional crosslinker, the crosslinker is, in an embodiment, a low molecular weight molecule (having a molecular weight below 2.5 kDa, in particular between 100 Da and 2.5 kDa, in particular between 500 Da and 2.0 kDa, in particular between 1 kDa and 1.5 kDa) comprising at least two reactive groups chosen independently from each other from the group consisting of methacryloyl, methacrylate, methacrylamide, acryloyl, thiol, vinyl, vinylene, allyl, alkynyl, epoxide, hydroxyl and conjugated dienyl groups. The presence of any of such groups allows the crosslinker to crosslink prepolymer molecules by the same chemistry as by which the individual prepolymer molecules are inherently crosslinked to each other.

In an embodiment, the crosslinker comprises two identical functional groups. In another embodiment, the crosslinker comprises at most one thiol group. In the latter or another embodiment, the crosslinker comprises only a thiol group if at least one functional group of the composition is chosen from the group consisting of methacryloyl, methacrylate, methacrylamide, acryloyl, vinyl, vinylene, allyl, alkynyl, epoxide, hydroxyl and conjugated dienyl groups, i.e. if at least one functional group is present that is not a thiol group. This facilitates the crosslinking properties of the crosslinker since the generation of disulfide bonds is suppressed to a significant extent or completely avoided.

In an embodiment, the composition comprises 30 to 95 percent by weight, in particular 35 to 90 % by weight, in particular 40 to 85 % by weight, in particular 45 to 75 % by weight, in particular 50 to 70 % by weight, in particular 55 to 65 % by weight, in each case based on the weight of the photocurable prepolymer, of the solvent.

In the same or another embodiment, the composition comprises 0.5 to 5 % by weight, in particular 1 to 4.5 % by weight, in particular 1.5 to 4 % by weight, in particular 2 to 3.5 % by weight, in particular 2.5 to 3 % by weight, in each case based on the weight of the photocurable prepolymer, of the photoinitiator.

In the same or another embodiment, the composition comprises 0 to 100 % by weight, in particular 5 to 95 % by weight, in particular 10 to 90 % by weight, in particular 15 to 85 % by weight, in particular 20 to 80 % by weight, in particular 25 to 75 % by weight, in particular 30 to 70 % by weight, in particular 35 to 65 % by weight, in particular 40 to 60 % by weight, in particular 45 to 55 % by weight, in each case based on the weight of the photocurable prepolymer, of functionalized poly($\varepsilon$-caprolactone) (PCL) bearing a third functional group. The embodiments relating to PCL explained above can be particularly well combined with this embodiment.

In the same or another embodiment, the composition comprises 0 to 0.5 % by weight, in particular 0.01 to 0.4 % by weight, in particular 0.05 to 0.3 % by weight, in particular 0.1 to 0.2 % by weight, in each case based on the weight of the photocurable prepolymer, of a light-absorbing colorant. The embodiments relating to the colorant explained above can be particularly well combined with this embodiment.

In the same or another embodiment, the composition comprises 0 to 20 % by weight, in particular 1 to 15 % by weight, in particular 2 to 12 % by weight, in particular 3 to 10 % by weight, in particular 4 to 8 % by weight, in particular 5 to 7 % by weight, in each case based on the weight of the photocurable prepolymer, of a crosslinker. The embodiments relating to the crosslinker explained above can be particularly well combined with this embodiment.

The photocurable composition as herein described is particularly appropriate to be used as resin (sometimes also referred to as ink) for manufacturing a three-dimensional object by 3D printing. For this purpose, the resin is used as a reservoir for three-dimensional structures, wherein the structures are finally formed by irradiating the resin with light having an appropriate wavelength (either in the visible or UV wavelength range or less often in the infrared wavelength range) so as to initiate a polymerization reaction by which polymerized, stable structures are formed in the light irradiated areas, wherein the resin in the non-irradiated areas remains liquid and can be easily removed.

In an embodiment, the three-dimensional object to be manufactured serves as scaffold for growing cells or tissue on it.

In an aspect, the present invention relates to a method for manufacturing a three-dimensional object from a resin by 3D printing the resin. Thereby, the method comprises the following steps:

a) providing the resin onto a carrier or a layer already formed from the resin,

b) illuminating those sections of the resin in a defined layer that are intended to crosslink,

c) letting automatically crosslink the illuminated sections of the resin, and

d) repeating steps a) to c) for a desired amount of layers,

Thereby, the resin comprises i) at least one photocurable prepolymer chosen from the group consisting of functionalized gelatin bearing a first functional group and functionalized decellularized extracellular matrix bearing a second functional group, ii) at least one solvent, the solvent being formamide, and iii) at least one photoinitiator.

In an embodiment, the method is carried out at a temperature lying in a temperature range of 15 °C to 37 °C, in particular of 20 °C to 35 °C, in particular of 22 °C to 32 °C, in particular of 25 °C to 30 °C. A temperature range of at most 32 °C is particularly appropriate if a functionalized, particularly star-shaped PCL with a molecular weight of around 2 kDa (in particular between 1.5 and 2.5 kDa) is present in the composition, since 32 °C is the melting point of such PCL prepolymers. Generally, a working temperature above 37 °C is not as favorable as lower working temperatures since some of the native structural elements of the individual components of the composition might lose their structural and/or functional integrity at those temperatures being higher than the native (body) temperature.

All embodiments of the described composition can be combined in any desired way and can be transferred either alone or in any desired combination to the described use and the described method. Likewise, the embodiments of the described use can be combined in any desired way and can be transferred either alone or in any desired combination to the described composition and the described method. Furthermore, the embodiments of the described method can be combined in any desired way and can be transferred either alone or in any desired combination to the described composition or the described use.

Further details of aspects of the present invention will be described with respect to exemplary embodiments and accompanying Figures. In the Figures:

Figure 1A     shows a schematic representation of a photochemically initiated radical crosslinking of a protein-based hybrid resin during SLA printing;

Figure 1B     shows a workflow of a typical digital light processing SLA printing process as illustrated for a small intestine tissue scaffold starting with a CAD model, slicing it into horizontal layers for photoprojection, and finally printing it layer by layer;

Figure 2A     shows a representative $^1$H NMR spectrum of porcine GelMA in $D_2O$ with a proton peak assignment of its methacrylamide group;

Figure 2B     shows a representative $^1$H NMR spectrum of star-shaped PCL-MA in $CDCl_3$ with a peak assignment of its methacrylate group;

Figure 3A     shows the mean residue ellipticity [Θ] of proteins at a concentration of 0.1 mg/mL in $NaHCO_3$ buffer at 20 °C measured by CD analysis in the far UV regime revealing the secondary structure of gelatin, GelMA, and GelMA in formamide for porcine gelatin (40 wt-% porcine GelMA before dissolving in the buffer);

Figure 3B     shows the mean residue ellipticity [Θ] of proteins at a concentration of 0.1 mg/mL in $NaHCO_3$ buffer at 20 °C measured by CD analysis in the far UV regime revealing the secondary structure of gelatin, GelMA, and GelMA in formamide for fish gelatin (45 wt-% fish GelMA before dissolving in the buffer);

Figure 4A     shows representative temperature-dependent viscosity curves of different resins before crosslinking, measured by a shear rheometer, wherein the concentration of a colorant in the resins was 0.1 wt-%;

Figure 4B     shows crosslinking curves of the resins of Figure 4A at 32 °C when exposed to visible light of a digital light processing SLA printer;

Figure 5A     shows the water contact angle of wet 3D samples printed from the resins of Figure 4A, wherein the error bars represent the standard deviation, n = 3;

Figure 5B     shows the swelling ratio of wet 3D samples printed from the resins of Figure 4A, wherein the error bars represent the standard deviation, n = 3;

Figure 5C     shows the elastic modulus of wet 3D samples printed from the resins of Figure 4A, wherein the error bars represent the standard deviation, n = 3;

| Figure 5D | shows the albumin permeability of wet 3D samples printed from the resins of Figure 4A, wherein the error bars represent the standard deviation, n = 3; |
|---|---|
| Figure 5E | shows the degradation of wet 3D samples printed from the resins of Figure 4A in collagenase solution at 37 °C , wherein the error bars represent the standard deviation, n = 3; |
| Figure 6A | shows metabolic activity of Caco-2 cells (initial seeding density 31,250/cm$^2$) on 3D printed materials from the resins of Figure 4A, measured by a PrestoBlue assay and normalized to the cell activity on tissue culture polystyrene (TCPS) samples at day 7; the error bars represent standard deviation of the samples, n = 3; |
| Figure 6B | shows brightfield microscope images of the Caco-2 cells of Figure 6A confirming the cell adhesiveness and the proliferation on the 3D printed material surfaces; |
| Figure 7 | shows in the first column fluorescently stained ZO-1 proteins of tight junctions (originally red) and in the second and third column SEM images of microvilli structures of Caco-2 cells at day 10 on 3D printed films of A) FGelMA/PCL-MA (first line) and B) PGelMA/PCL-MA (second line) as well as on C) TCPS control (third line); |
| Figure 8 | shows a CAD model of a human villi-containing intestinal scaffold (panel A) and images of wet 3D printed intestinal scaffolds made from fish GelMA (FGelMA) resin (panel B), porcine GelMa (PGelMA) resin (panel C), PCL-MA resin (panel D), FGelMA/PCL-MA resin (panel E), and PGelMA/PCL-MA resin (panel F); the scale bar represents 500 $\mu$m; |
| Figure 9A | shows a $^1$H NMR spectrum of the formulated dECM after methacrylation; |
| Figure 9B | shows the mean residual ellipticity of dECM in NaHCO$_3$ buffer at 20 °C measured by far-UV CD analysis revealing its secondary structure before and after methacrylation; |
| Figure 9C | shows a temperature-dependent shear viscosity of the dECM-MA/PCL-MA resin; and |
| Figure 9D | shows a workflow from a decellularized rat liver via lyophilized dECM-MA powder to a wet 3D printed annular dECM-MA/PCL-MA hydrogel. |

The Figures will be explained in the following in more detail with respect to exemplary embodiments.

## Materials and methods

### Materials

ε-caprolactone monomer (ε-CL, Alfa Aesar, 99 %) was dried over CaH2 (Acros Organics, 93 %) and distilled under reduced pressure before polymerization. Tin(II) 2-ethylhexanoate (Sn(Oct)$_2$, Aldrich, 92.5 %), di(trimethylolpropane) (diTMP, Aldrich, 97 %), methacrylic anhydride (Aldrich, 94 %), gelatin from cold water fish skin (60 kDa, Aldrich), gelatin from porcine skin (gel strength 300, type A, 50-100 kDa, Aldrich), formamide (99.5 %, Roth), ethyl(2,4,6-trimethylbenzoyl) phenylphosphinate (also known as Lucirin TPO-L) photoinitiator (Fluorochem), Orasol Yellow 2RLN dye (Kremer Pigmente), 2,4,6-trinitrobenzene sulfonic acid (TNBSA), pepsin from porcine gastric mucosa (Sigma), and lyophilized collagenase class I and II from *Clostridium histolyticum* (NB 4 Standard grade min 0.1 U/mg, Serva) were used as received. Human colorectal adenocarcinoma Caco-2 cells were purchased from ATCC (HTB-37™). Minimal essential medium (MEM, Gibco), fetal bovine serum (FBS, Biochrom), penicillin-streptomycin (Life Technologies), sodium pyruvate (100 mM, Gibco), non-essential amino acid solution (100X, Sigma), PrestoBlue assay (Thermo Fisher), glutaraldehyde solution (50 % in water, Aldrich), hexamethyldisilazane (Aldrich), bovine serum albumin (BSA, Sigma), rabbit anti-human ZO-1 antibody (Abcam), goat anti-rabbit IgG Alexa Fluor 647 antibody (Abcam), 4,6-diamidin-2-phenylindol (DAPI, Thermo Fisher), and phalloidin-Atto 647 (Sigma) were used in cell culture studies as received or diluted.

### Synthesis of photocrosslinkable PCL-, gelatin-, and dECM-based macromers

A four-armed hydroxyl-terminated PCL oligomer with a targeted molecular weight of 2,000 g/mol was synthesized by ring-opening polymerization of ε-caprolactone monomers at 140 °C for 24 h under an argon atmosphere using di(trimethylolpropane) as the initiator and 0.02 mol % $Sn(Oct)_2$ as the catalyst. Photocrosslinkable PCL-MA macromer was synthesized by functionalizing the PCL oligomer (10 g) with methacrylic anhydride following a previous protocol (Elomaa et al., 2011). After methacrylation, PCL-MA was precipitated in cold isopropanol, isolated, and dried under reduced pressure to yield 11.5 g of the colorless waxy polymer. Fish and porcine-derived gelatin were methacrylated following a sequential protocol from Lee et al., 2015. Briefly, gelatin (10g) was first dissolved in sodium carbonate-bicarbonate buffer of pH 9.7 (10 % wt/v) at 50 °C, after which methacrylic anhydride (0.1 mL per 1 g of gelatin) was added sequentially during 3 h with 1 N of NaOH to adjust the pH to 9. After the methacrylation, pH was adjusted to 7.4 and the biopolymer was dialyzed with 3.5 kDa tubing against distilled water for 2 days. The biopolymer was finally lyophilized, yielding 9.5 g of fish GelMA (FGelMA) as a lightly yellow powder and 9.7 g of porcine GelMA (PGelMA) as a colorless powder.

Decellularization of rat livers was performed according to a previously established protocol (Struecker et al., 2015). All animal work was performed in accordance with local law and approved by the State Office of Health and Local Affairs (LAGeSo, Berlin, Germany; Reg. No. L0421/12) Briefly, the organs were explanted as a part of a teaching program and preserved in -80 °C in Ringer's solution until use. Prior to decellularization, they were thawed overnight in 4 °C and subsequently were first perfused with PBS for 10 minutes followed by 1 % Triton X-100 for 90 minutes and 1 % SDS for 90 minutes. Finally, the organs were rinsed with PBS overnight. The resulting colorless dECM lobes were solubilized following a protocol from Rothrauff et al., 2018. First, the frozen dECM was grinded with a mortar and a pestle, after which the powder was digested in acidic pepsin solution (1 mg/mL in 0.01 N HCl) at room temperature for 3 days. The dECM solution was then adjusted to pH 7.4 by addition of 1N NaOH. After lyophilization, the dECM powder (0.75 g) was dissolved in sodium carbonate-bicarbonate buffer of pH 9.7 (10 % wt/v) at RT, after which methacrylic anhydride (0.3 mL per 1 g of dECM) was added sequentially during 3 h with 1 N of NaOH to adjust the pH to 9. After continuing the methacrylation for 1 day at RT, the pH was adjusted to 7.4 and the biopolymer was dialyzed with 3.5 kDa tubing against distilled water for 3 days and lyophilized, yielding 0.70 g of a fine colorless powder.

**Formulation of the resins and 3D printing with DLP stereolithography**

To 3D print the new materials, different resin compositions were formulated in formamide as shown in Table 1, including hybrid resins of PCL-MA with fish GelMA (FGelMA/PCL-MA) or porcine GelMA (PGelMA/PCL-MA), pure resins of 100 wt-% FGelMA or 100 wt-% PGelMA, and neat (pure) PCL-MA as control. PCL-MA resin did not require use of diluents to obtain a suitable viscosity for SLA, while lyophilized GelMA powders were dissolved in formamide at 37 °C before mixing with PCL-MA (70/30 weight ratio without solvent) or printing as such to adjust an appropriate viscosity range for SLA. 0.10 wt-% of Orasol Yellow dye was added to the resins to increase the extinction coefficient and thereby decrease the penetration depth of light, allowing the highest control over photocrosslinking. Furthermore, a dECM-MA/PCL-MA (70/30 wt-%) resin was developed by first dissolving the lyophilized dECM-MA powder in formamide (50 wt-%) and then blending the solution with PCL-MA dissolved in benzyl alcohol at the same weight ratio (50 wt-%).

*Table 1. The polymer composition of resins for SLA*

| Resin | GelMA or dECM-MA (wt-% of polymer) | PCL-MA (wt-% of polymer) | Formamide (wt-% of resin) | Benzyl alcohol (wt-% of resin) |
|---|---|---|---|---|
| PGelMA | 100 | 0 | 55 | 0 |
| FGelMA | 100 | 0 | 50 | 0 |
| PCL-MA | 0 | 100 | 0 | 0 |
| PGelMA/PCL-MA | 70 | 30 | 51 | 0 |
| FPGelMA/PCL-MA | 70 | 30 | 46 | 0 |
| dECM-MA/PCL-MA | 70 | 30 | 35 | 15 |

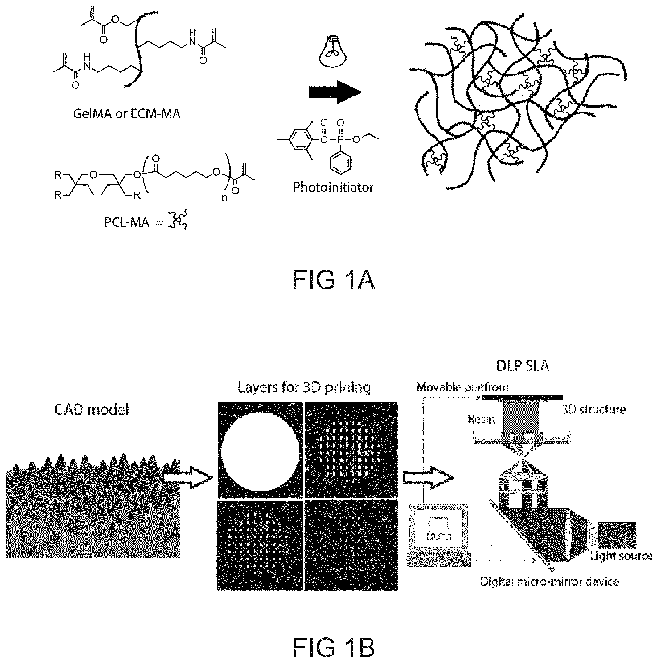

Before 3D printing, all resins were mixed with 2 wt-% of ethyl(2,4,6-trimethylbenzoyl) phenylphosphinate photoinitiator. To characterize physicochemical and biological properties of the new resins, i.e swelling, mechanical properties, degradation, permeability, and cell response, first planar disc-shaped samples (d = 5 or 12 mm) were 3D printed. After physicochemical characterization, the suitability of the resins for use in 3D small intestine tissue scaffolds was evaluated by CAD modeling and printing disc-shaped tissue scaffolds (d =12 mm) with villi-mimicking spikes (height = 1 mm) on their top surface. The CAD models were designed using Rhinoceros 5 software and sliced with Creation Workshop software. The DLP SLA printer (Titan 2 from Kudo3D, Taiwan) was equipped with a UV filtered projector (Acer Full HD 1080) that projects 1920x1080 pixels of visible light onto a bottom of a resin reservoir to crosslink a desired layer thickness, in the currently described experiments 50 $\mu$m (Figure 1). The temperature inside the printing hood was controlled with an additional thermostat and a heater (IncuKit™ Mini, Incubator Warehouse). After printing, uncrosslinked macromer as well as formamide solvent were removed from the samples by extraction with an isopropanol/acetone (3:1) mixture for 24 h and then milli-Q water for 7 days at RT with repeated water changes.

**Physicochemical characterization of materials**

$^1$H NMR spectra were recorded with a Jeol ECX 400 MHz or Bruker Avance 700 MHz NMR spectrometer. Samples were dissolved in deuterated chloroform ($CDCl_3$) or water ($D_2O$) (10 mg/mL), the spectrum was acquired in a 5 mm NMR tube at RT. The conversion of primary amine groups of lysine residues in gelatin and solubilized dECM into methacrylamide groups was accessed indirectly by quantifying the remaining free primary amine groups using a colorimetric TNBSA assay. The lyophilized gelatin- or solubilized dECM-based samples, before and after modification, were dissolved in sodium bicarbonate buffer ($NaHCO_3$, pH 8.5, 0.1 M) at a concentration of 200 $\mu$g/mL, and the protein solutions (0.5 mL) were then mixed with 0.01 % (w/v) TNBSA (0.25 mL) in buffer. The solutions were then incubated at 37 °C for two hours, after which 10 % SDS (0.25 mL) and 1 M HCl (0.125 mL) was added. The absorbance A was measured at 335 nm using an ultraviolet (UV) spectrometer (Cary 8454, Agilent Technologies). The degree of methacrylamidation was calculated from the ratio of absorbance of the modified to the unmodified sample using Equation (1):

$$1 - A_{GelMA} / A_{gelatin} \times 100\ \% \qquad (1)$$

Secondary structure of gelatin and solubilized dECM before and after methacrylation was analyzed with far-UV circular dichroism (UV CD) spectroscopy by dissolving the respective proteins in 0.1 M $NaHCO_3$ buffer at 0.1 mg/mL. Samples were measured using a Jasco 815 CD spectrometer and 1 mm quartz cuvette under N2 atmosphere at 20 °C. To study the effect of formamide on the secondary structure of GelMA, GelMA/formamide resin was dissolved in buffer with the polymer concentration of 0.1 mg/mL. In that case, the blank was the corresponding amount of formamide (0.12 for FGelMA and 0.15 mg/mL for PGelMA) dissolved in buffer, while otherwise the blank was neat buffer. For calculating mean residue ellipticity, the average molecular weight of the amino acids was chosen to be 120 g/mol. Shear viscosity of the printing resins was analyzed using a Kinexus pro+ rotational rheometer (Malvern Panalytical) with a 20 mm/1° cone plate geometry. During the measurements the temperature was ramped between 20-40 °C with a constant shear rate of 1 1/s. Crosslinking kinetics of the resins were studied by photocrosslinking a constant amount of the resin with the DLP SLA printer for varied exposure times. Derived from Beer-Lambert law, the cure depth $C_d$ ($\mu$m) depends on the light energy dose E ($mJ/cm^2$) and thereby on the exposure time t (s) via Equation (2):

$$C_d = D_p ln(E) - D_p ln(E_c) = D_p ln(t) + D_p ln(I) - D_p ln(E_c) \qquad (2)$$

where I($mW/cm^2$) is the intensity of the light, $D_p$ is the penetration depth of light ($\mu$m), and $E_c$ ($mJ/cm^2$) is the minimum energy required for gelling. When the cure depth was drawn as a function of exposure time, working curves of the resins were obtained.

Hydrophobicity of the wet crosslinked samples (d = 5, h = 2 mm) was accessed with water contact angle measurements (DataPhysics Contact Angle System OCA) applying 2 $\mu$L milli-Q water on the flat substrates. The contact angles of the profile of three replicate sessile drops on a sample were analyzed with the software package SCA202 version 3.12.11 applying an ellipse-fitting model and averaged. To further measure the swelling ratio of the crosslinked materials, the same samples were weighed and immersed in distilled water for 24 h to reach the swelling equilibrium, after which the samples were dried overnight at 60 °C and weighed again. By using wet weight ($m_{sw}$) and dry weight ($m_d$) of the samples and the density values of 1.0 g/ml for water ($\rho_s$) and 1.1 g/ml as a rough estimate for the crosslinked polymer ($\rho_p$), the degree of swelling Q was calculated using Equation (3):

$$Q = 1 + \rho_p / \rho_s \times (m_{sw}/m_d - 1) \qquad (3)$$

To study enzymatic degradation of the 3D printed materials, mass loss of the crosslinked samples (d = 5, h = 2 mmm) was monitored by incubating the samples for predetermined time in collagenase solution (0.5 U/ml in PBS) at 37 °C and measuring the weight of the samples after rinsing them with PBS and drying overnight at 60 °C. After drying, the remaining mass was divided by the initial mass to obtain the remaining mass percentage. Mechanical properties of the 3D printed materials were studied with an oscillating mode of the rotational rheometry (Kinexus pro+) using an 8 mm parallel plate geometry. First, the viscoelastic regime of wet samples (d = 8 mm, h = 2 mm) was determined with an amplitude sweep from 0.1 to 50 % strain at 1 Hz, after which the elastic modulus was measured with a frequency sweep from 0.1 to 10 Hz at 37 °C using a 0.1 % strain and a 0.1 N initial normal force.

**Albumin permeability test**

To study albumin permeability of the crosslinked materials, 3D printed films (d = 12 mm, h = 0.5 mm) were glued on Millicell® cell culture inserts (A = 0.6 cm$^2$, Merck) after removal of the original porous membrane. Unmodified inserts with a polycarbonate membrane (0.4 $\mu$m pore size) were used as controls. After gluing and pre-wetting in PBS, the inserts were placed into wells with 600 $\mu$L of PBS. In the beginning of the permeability test, 400 $\mu$L of BSA solution (5 mg/mL in PBS) was added inside the inserts and incubated at 37 °C. Two replicate 100 $\mu$L aliquots were taken at predetermined times from the receiver chambers and were replaced with PBS. The albumin concentration of the aliquots was analyzed by reading their UV absorption at 280 nm with a plate reader (Tecan Infinite M200) and using a calibration curve of the same albumin compound. The permeability coefficient $P$ (cm s$^{-1}$) was obtained using Equation (4):

$$P = dQ/dt \times 1/(Ac_0) \quad (4)$$

where $dQ/dt$ is the steady-state flux ($\mu$g/s), A is the surface area of the insert (cm$^2$) and $c_0$ is the initial concentration of albumin in the donor chamber ($\mu$g/cm$^3$).

**Cell culture and cell proliferation**

Caco-2 cells were cultured in minimum essential medium (MEM) mixed with 20 % FBS, 1 % non-essential amino acid solution, 1 % sodium pyruvate, and 1 % penicillin/streptomycin antibiotic solution under standard conditions (5 % $CO_2$ , 37 °C, 95 % humidity). Before cell seeding, 3D printed films (d = 5 mm, h = 0.2 mm) were treated with 70 % ethanol for 30 min followed by immersion in PBS (3 times for 15 min). $1 \times 10^4$ cells per sample were seeded on the samples, and at predetermined times, the metabolic activity of cells was measured using a colorimetric PrestoBlue cell viability assay. Before the assay, the samples were transferred to a new well plate, after which 100 $\mu$L of fresh medium mixed with 10 $\mu$L of PrestoBlue solution was added into each well. After incubation for 1 h, the colored medium from each well was transferred to a new plate, and the absorbance was measured using a plate reader (Tecan Infinite M200) at 570 nm and 600 nm. In the end of the experiment, the absorbance values were normalized to the 100 % control values obtained from cells grown on TCPS samples at day 7, and the average of three individual experiments with triplicate samples were then calculated.

**SEM imaging of a microvilli structure and fluorescence imaging of tight junctions**

Differentiation of Caco-2 cells into polarized phenotype was monitored by imaging their brush border by scanning electron microscopy (SEM, Hitachi SU8030, 15 kV) and by staining of their tight junctions. For SEM imaging, the samples from the cell proliferation test were cultured until day 10 and then rinsed with PBS, after which the cells were fixed with 2.5 % glutaraldehyde/PBS solution for 3 h. After rinsing three times with Milli-Q water, the samples were dehydrated in an ethanol series (50-70-90-100-100 %) for 10 min each. Finally, the samples were dried with a hexamethyldisilazane/ethanol (1:1) solution and pure hexamethyldisilazane for 10 min each. Before SEM imaging, samples were made electrically conducting by sputter coating them with a thin gold layer.

To make the tight junctions visible, zonula occludens (ZO-1) tight junction proteins expressed on differentiated Caco-2 cells were stained with ZO-1 antibody. First, the samples from the cell proliferation test at day 10 were rinsed with PBS, after which the cells were fixed with cold methanol for 5 min at 4 °C. After evaporating the methanol for 15 min, the samples were rinsed again with PBS and immersed for 2 min at RT in washing buffer containing 0.05 % Tween 20 in PBS. Rabbit anti-human ZO-1 primary antibody was diluted in buffer containing PBS/0.05 % Tween 20/1 % BSA with the mixing ratio of (1:250) and goat anti-rabbit IgG AlexaFluor 647 secondary antibody with the ratio of 1:400. The cell-containing samples were first immersed in the primary antibody solution for 1h at RT, after which they were washed three times with the washing buffer and then immersed in the secondary antibody solution for 30 min at RT in dark. After washing the samples again three times with the washing buffer, the samples were mounted on a glass slide and enveloped with cover slips. Fluorescence images were taken with a fluorescence microscope (Zeiss Axio Observer Z1) at 644/669 nm to visualize tight junctions.

**Statistical analysis**

For the statistical analysis of the cell proliferation tests, three independent experiments were conducted with triplicate samples, and the statistical significance was determined using a one-way ANOVA test followed by Tukey's post hoc test ($p < 0.05$) provided by Originlab 2019 software.

**Results**

**Structural characterization of fish and porcine GelMA and PCL methacrylate**

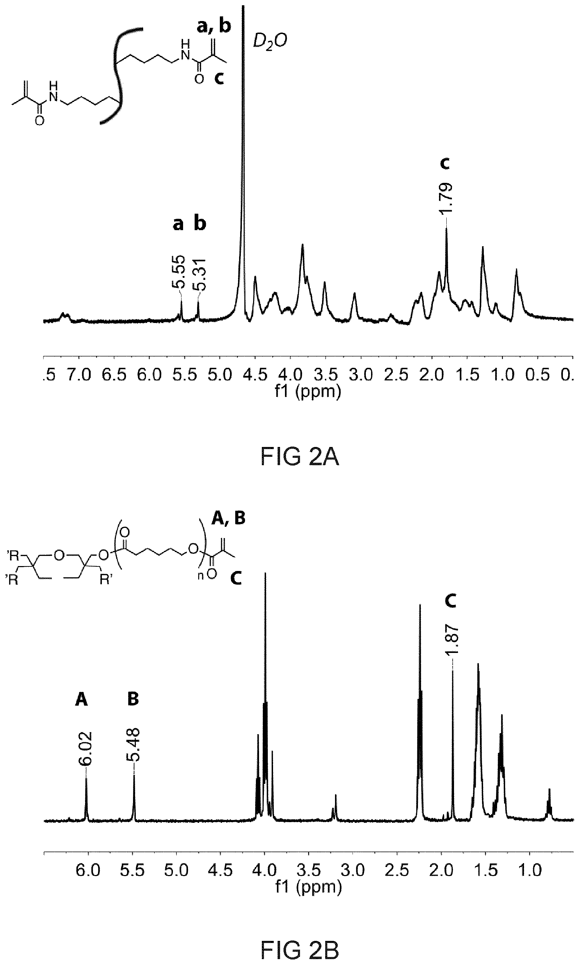

The star shaped, hydroxyl-terminated PCL was synthesized via ring-opening polymerization, resulting in a molecular weight of 2,030 g/mol according to $^1$H NMR spectroscopy. The PCL oligomer and commercial gelatin were then functionalized with methacrylic anhydride to obtain photocrosslinkable macromers. Successful methacrylation of PCL as well as both fish and porcine gelatin was confirmed with $^1$H NMR analysis. As the major methacryloyl group in GelMA is known to be methacrylamide, while methacrylate represents only less than 10 % of all substitutions, the inventors focused on detecting the methacrylation of primary amine groups of gelatin. The $^1$H NMR spectrum of GelMA (Figure 2A) revealed the disappearance of the gelatin proton peak at 2.86 ppm attributed to the CH2 group located next to an unmodified pending NH2 group and the appearance of new peaks a, *b*, c (5.55, 5.31, 1.79 ppm) attributed to the methacrylamide end group appeared. Figure 2A shows this representatively for porcine GelMA, but it was observed identically for fish GelMA as well. Methacrylation of PCL was confirmed by the disappearance of the peak at 3.63 ppm attributed to the last CH2 group of a PCL arm and appearance of the peaks A, *B, C* (6.02, 5.48, 1.87 ppm) attributed to the methacrylate end group (Figure 2B). The complete disappearance of the peaks attributed to the non-reacted chain ends upon methacrylation in the $^1$H NMR spectra indicated a high degree of functionalization both for gelatin and PCL. However, as the complex structure of gelatin polypeptides may hinder the precise quantitative detection of methacrylamides by $^1$H NMR spectroscopy, the ratio of primary amines in gelatin was further quantified before and after methacrylation by a colorimetric TNBSA assay. The latter indicated a degree of methacrylation of 99 % for both fish and porcine gelatin, which was in good agreement with the NMR analysis.

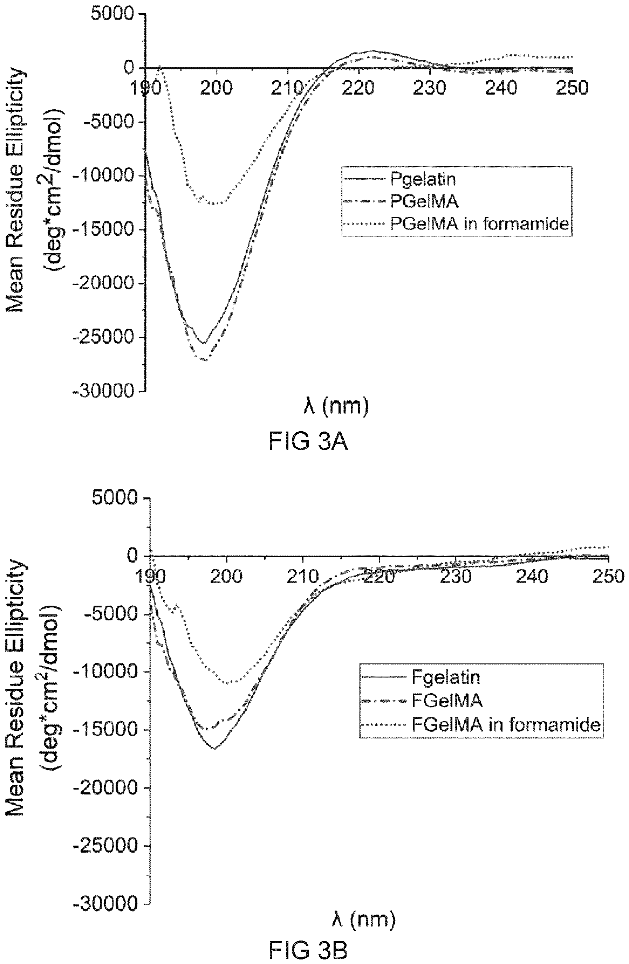

The effect of methacrylation and formamide solvent on the secondary structure of gelatin was studied by a far-UV CD spectroscopy. The CD curves in Figure 3 indicate the unaffected secondary structure of gelatin upon methacrylation. Both before and after methacrylation, porcine gelatin (Pgelatin) showed a negative band at 198 nm assigned to its random coil conformation and the small positive band at 221 nm assigned to its residual triple helical structure. Fish gelatin (Fgelatin) did not show the positive band, thus indicating its irregular random coil structure. GelMa samples in the presence of formamide did not show the positive band, suggesting denaturation of the triple helix tertiary structure of PGelMA in formamide.

**Temperature-dependent viscosity and crosslinking kinetics of the resins**

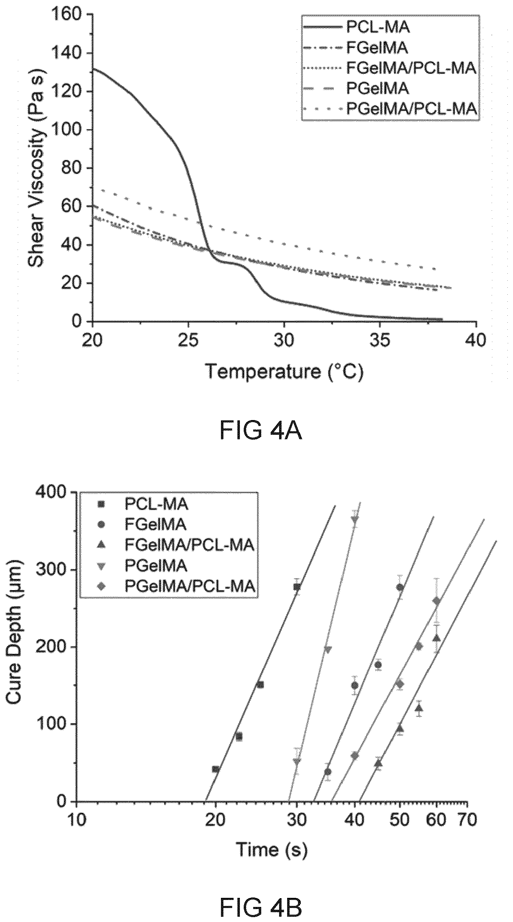

To achieve a high resolution in DLP SLA printing, precise control over the viscosity and cure depth of the resin is critical. The viscosity of the resin can be controlled by adjusting the printing temperature. The optimal printing temperature of the neat PCL-MA resin was evaluated by studying its shear viscosity in the range of 20 to 40 °C. Analogously, the GelMA resins were investigated but as solution in formamide at a concentration of 40 wt-% for the porcine and 45 wt-% for the fish sample. As Figure 4A shows, the neat PCL-MA had a drastic decrease in viscosity because of melting, while the viscosity of the GelMA solutions decreased more steadily. The addition of PCL-MA into the hybrid resin slightly increased the viscosity of PGelMA while viscosity of FGelMA remained the same. Based on the viscosity curves, the inventors chose for 3D printing the lowest temperature where all the resins were liquid, that is 32 °C. To evaluate the time needed for crosslinking targeted 50 $\mu$m layers while 3D printing at 32 °C, the working curves of the resins were drawn to show the influence of the exposure time on cure thickness (Figure 4B). The PCL-MA resin required the shortest exposure time (20 s) for crosslinking a 50 $\mu$m layer, while the GelMA-containing resins required a longer exposure time. PGelMA required a slightly shorter time (30 s) than FGelMA (35 s). GelMA/PCL-MA hybrid samples crosslinked most slowly: PGelMA/PCL-MA required 38 s and FGelMA/PCL-MA 45 s for crosslinking a 50 $\mu$m layer. To ensure good adhesion between successive layers, the respective exposure time was increased by 10 % for the 3D printing.

**Swelling, mechanical properties, permeability, and enzymatic degradation of 3D printed materials**

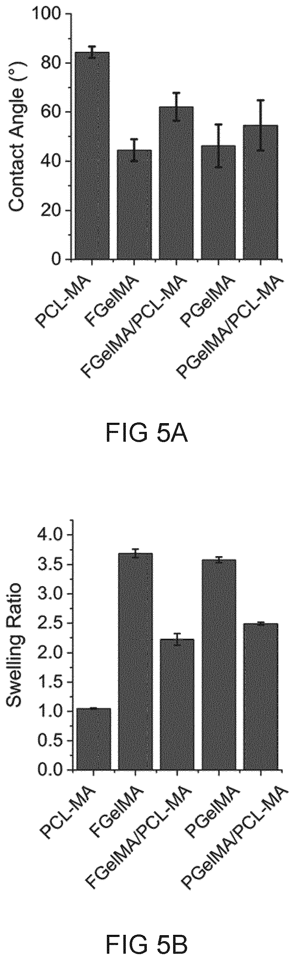

As the new hybrid resin combined two very different materials, the inventors thoroughly characterized the physical properties of the 3D printed materials. The hydrophilicity of the material that affects its swelling and cell adhesion capacity was assessed by the water contact angle. Figure 5A demonstrates that addition of GelMA into PCL-MA decreased the water contact angle from 84° $\pm$ 2° (PCL-MA) to 62° $\pm$ 6 (FGelMA/PCL-MA) and 55° $\pm$ 10° (PGelMA/PCL-

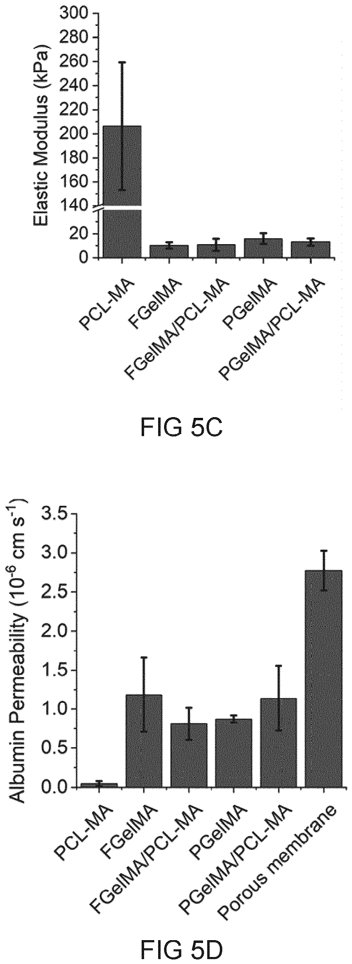

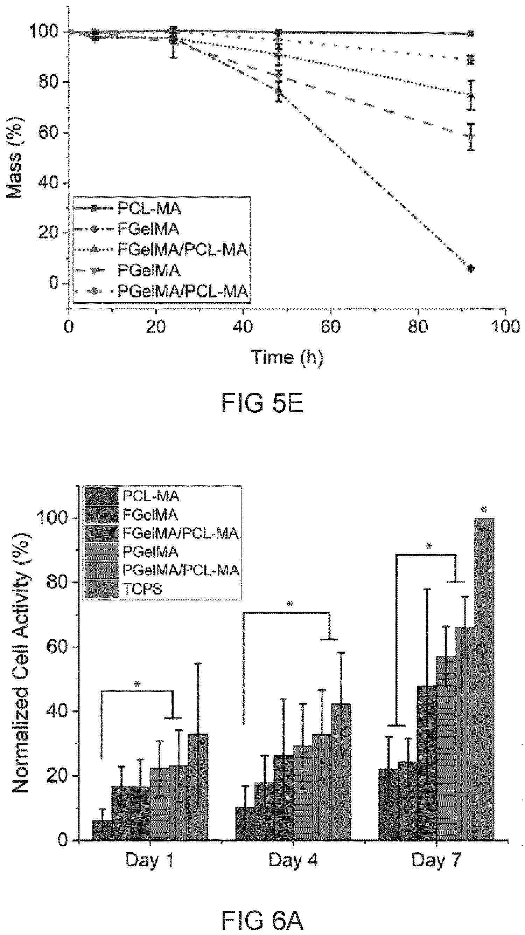

MA), meaning that the surface energy of the material and the interfacial tension increased with the GelMA addition. The increased hydrophilicity resulted also in a 2-3.5-times higher swelling ratio of GelMA-containing samples compared to PCL-MA that did not swell at all (Figure 5B). The rheometric studies of viscoelastic properties of the 3D printed materials revealed that the wet GelMA-containing samples had significantly lower elastic modulus than the neat PCL-MA samples (Figure 5C). Fish and porcine GelMA had similar elastic moduli in the range of 10 kPa, and notably the addition of PCL-MA did not significantly influence their modulus. To evaluate permeability of the samples to macromolecules in cell culture medium, BSA was used as a model compound due to its large size (~66 kDa). Figure 5D shows that the GelMA-containing samples clearly had a higher permeability to albumin than neat PCL-MA samples, which virtually did not allow any albumin permeation. However, permeability of all gelatin-containing samples remained at about 30 % of the one of highly porous, track-etched polycarbonate membranes used a control. Furthermore, to ensure that crosslinking did not prevent enzymatic degradation of gelatin, mass loss of all the printed materials was examined in collagenase solution at 37 °C. Figure 5E indicates that PCL-MA samples did not degrade within 96 h, while GelMA-containing samples started to loose mass constantly after 20 h. FGelMA samples were fully degraded after 96 h, while PGelMA samples lost 42 % of their mass after 96 h. The addition of PCL-MA slowed down the degradation rate of both GelMA/PCL-MA hybrid materials compared to pure GelMA samples.

## Caco-2 proliferation and differentiation on 3D printed materials

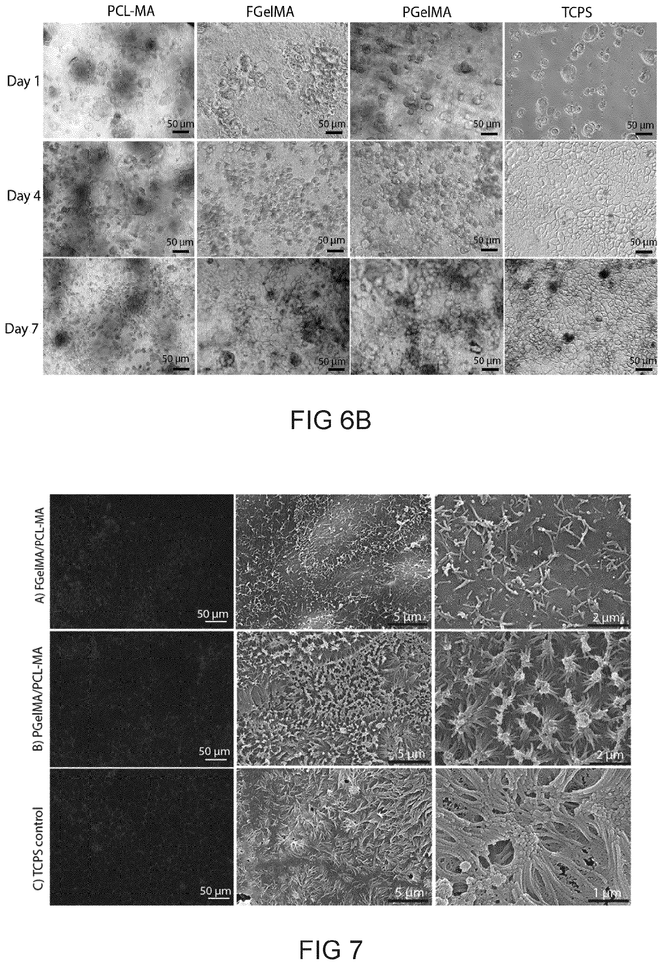

The cell adhesion and proliferation on the 3D printed materials were studied by measuring metabolic activity of Caco-2 cells on the material surface. The results of the PrestoBlue assay in Figure 6A exhibited an increased average metabolic activity of the cells from day 1 to day 7 on all the samples, which correlated to the cell proliferation. Cells proliferated significantly better on thePGelMA samples compared to the FGelMA samples, and the proliferation was significantly better on the hybrid PGelMA/PCL-MA samples compared to the neat PCL-MA samples. The brightfield microscopy images in Figure 6B illustrate the increase in the cell number on the samples and revealed the desired epithelial cell-like morphology of Caco-2 cells. However, non-transparent hybrid GelMA/PCL-MA samples did not allow brightfield microscopic imaging of the cells.

To further study the proliferation and enterocytic differentiation of Caco-2 cells on the non-transparent GelMA/PCL-MA hybrid materials, tight junctions of the cells were fluorescently stained after 10 days of cell culture. Immunofluorescent staining in Figure 7 revealed that both FGelMA/PCL-MA and PGelMA/PCL-MA hybrid materials supported the formation of tight junctions similar to the TCPS control. SEM images further revealed that Caco-2 cells formed desired microvilli structure on their apical membrane on day 10 on both hybrid materials, while on PGelMA/PCL-MA, the microvilli containing brush border appeared to be denser than on FGelMA/PCL-MA.

## 3D printing of intestinal tissue scaffolds

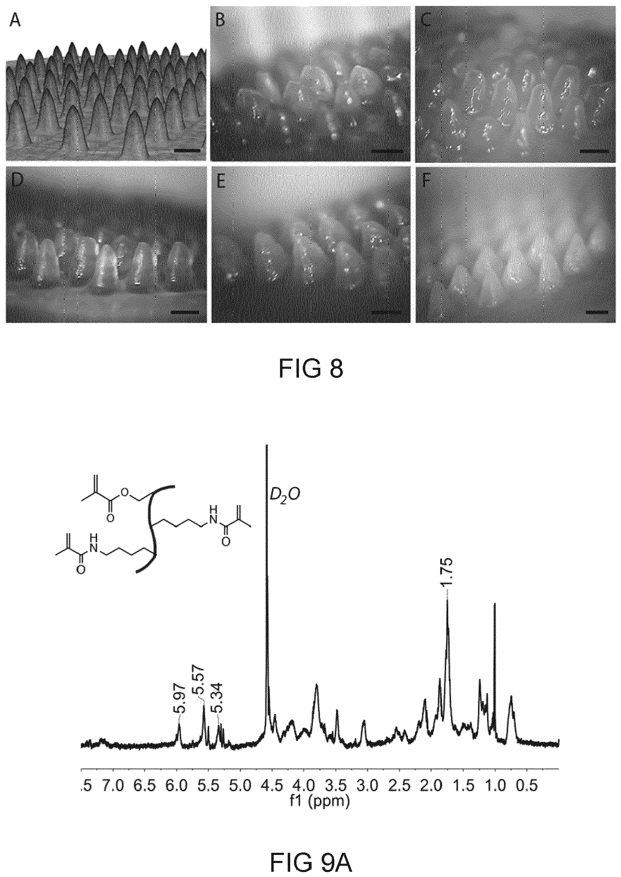

The GelMA/PCL-MA hybrid material was developed to enable the fabrication of more realistic *in vitro* intestinal tissue scaffolds. To 3D print the scaffolds mimicking a physiological villi structure, all the materials were formulated into photocrosslinkable resins. The crosslinking time was optimized for all the resins separately to obtain the best resolution as described above The neat PCL-MA scaffolds in Figure 8D resembled the best the CAD model shown in Figure 8A, while the pure FGelMA resin resulted in the lowest resolution with not fully evenly crosslinked, but still acceptable spikes (Figure 8B). However, the addition of 30 wt-% of PCL-MA into the GelMA resins remarkably improved the fidelity of the scaffolds compared to pure GelMA both in FGelMA/PCL-MA (Figure 8E) and PGelMA/PCL-MA (Figure 8F). Compared to pure GelMA resin, the hybrid resins therefore proved their improved suitability for 3D printing of tissue scaffolds with finely defined 3D features such as the intestinal lumen.

## dECM-MA/PCL-MA resin for 3D printing

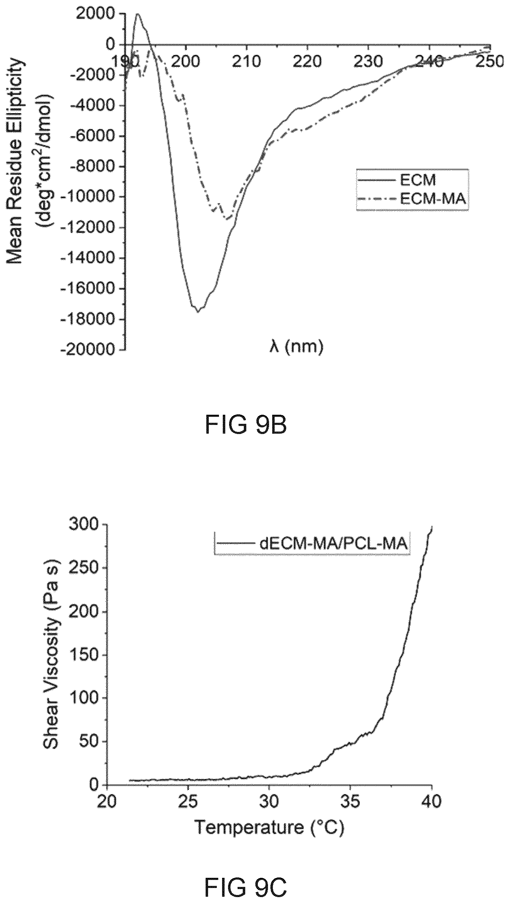

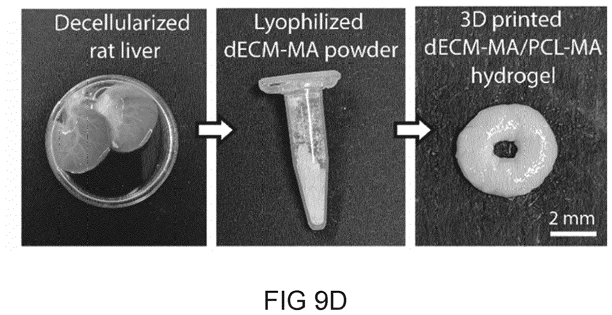

To highlight the transferability of the chosen approach to the use of native organ-derived material in SLA 3D printing, GelMA was replaced in the hybrid resin with methacrylated dECM. According to a $^1$H NMR analysis, the methacrylation of solubilized, rat liver-derived dECM led to an appearance of new peaks at 5.34, 5.57, and 5.97 ppm and at 1.75 ppm, which can be attributed to the new methacrylate and methacrylamide end groups of ECM macromolecules (Figure 9A). The TNBSA assay indicated 98 % conversion of free primary amines to methacrylamide groups. The far-UV CD analysis of dECM and dECM-MA in Figure 9B revealed no positive peak around 220 nm, indicating the lack of a tertiary triple-helix structure in the dECM proteins. To evaluate the suitability of the formulated dECM-MA/PCL-MA resin for SLA, its temperature-dependent viscosity was studied between 20 °C and 40 °C. Figure 9C reveals that the viscosity remained low until 32 °C, when the viscosity started to rapidly increase. The preliminary 3D printing of the resin with SLA in the absence of dye resulted in a soft hydrogel as illustrated in Figure 9D.

**Discussion**

Compared to 2D cell culture tissue models that poorly replicate native tissue anatomy, 3D printing offers a great way to fabricate more realistic tissue models with fine 3D features. However, to 3D print a tissue scaffold with a high fidelity, the material choice is critical. This is reemphasized by the fact that the material finally must support cell adhesion and proliferation on the printed scaffolds and allow efficient mass transport for nutrient supply and metabolic waste removal. In photocrosslinking-based SLA 3D printing, the resolution of the scaffolds strongly depends not only on the viscosity of the resin, but on the amount of reacting functional groups and the molecular weight of the polymer in the resin and the polymer concentration, since these factors determine the crosslinking density and crosslinking kinetics of the photocrosslinkable material. In the present invention, a high-resolution resin for SLA printing was developed that leads to a permeable material supporting growth and differentiation of intestinal epithelial cells. In the chosen approach, amongst others, a hybrid GelMA/PCL-MA (70/30 wt-%) resin was manufactured for use in a visible light DLP SLA printer to combine the desired cell adhesion and permeability properties of GelMA hydrogel with the superior printing properties of a low molecular weight PCL-MA resin. GelMAs were obtained with a high methacrylation degree via a pH-controlled reaction of porcine or fish gelatin with methacrylic anhydride. Maintaining the pH above 9 effectively prevented the ionization of free amino groups of gelatin and thereby ensured their availability for methacrylation, which was needed for successful methacrylation. Methacrylation of a star-shaped PCL with methacrylic anhydride in the presence of TEA yielded in a high degree of methacrylation at room temperature.

To obtain a homogenous 3D printable hybrid resin of GelMA and PCL-MA, formamide was used as a non-reactive diluent. When GelMA was dissolved in formamide, it lacked a gel-forming triple-helix structure, as confirmed by far-UV CD spectroscopy. This was a key for successful 3D printing, since temperature-dependent non-covalent gel formation due to spatially ordered triple helix structures of porcine GelMA stabilized by water in an aqueous solution is known to hinder its use in SLA. Use of formamide instead of water prevented the triple-helix formation due to strong hydrogen bonds between GelMA and formamide, and consequently, the non-covalent gel formation was hindered. Rheology studies revealed the slight decrease in viscosity of all the formulated GelMA-containing resins with the increasing temperature, even though the dependence on temperature was less pronounced compared to aqueous GelMA solutions studied elsewhere. Based on the viscosity curves, the 3D printing temperature was set to 32 °C, where the PCL-MA resin had lost its waxy appearance and all of the resins appeared as freely flowing liquids with a viscosity between 7 and 37 Pa s.

When photocrosslinked at 32 °C, the formulated GelMA resins polymerized more slowly than the PCL-MA resin. The faster crosslinking of PCL-MA was partly attributed to its closer proximity of the reacting methacrylate groups, as the reaction kinetics depends on the density of functional groups in the prepolymer. The PCL-MA had on average 1 kDa polymer chains between reactive double bonds, while in GelMA, the roughly estimated average chain length between methacrylamides was 2-2.5 kDa, assuming the lysine content of gelatin (50-100 kDa for Pgelatin, 60 kDa for Fgelatin) to be 3-4 wt-%. Furthermore, the less hindered macromolecular motion of PCL-MA chains at 32 °C could have contributed to the faster crosslinking kinetics as the glass transition temperature, where the molecular motion stops, is around -60 °C for PCL-MA and at the range of 80-90 °C for the amorphous region of gelatin.

The difference in crosslinking kinetics was important as it directly influenced the 3D printing resolution in SLA. As the formulated GelMA resins crosslinked more slowly than the neat PCL-MA resin, free radicals had more chance to diffuse and react outside of the area exposed to light, impairing thereby the resolution. The low molecular weight PCL-MA in turn photopolymerized fast and formed tightly crosslinked networks due to its short chains, resulting in a higher printing resolution. Besides the fast reaction kinetics, the low viscosity of the PCL-MA resin improved its resolution as it enabled free flow of the resin, preventing the layers from overcrosslinking during the 3D printing. The superior photocrosslinking kinetics of the neat PCL-MA resin translated into the GelMA/PCL-MA hybrid resins as their 3D printing resulted in remarkably enhanced resolution and fidelity compared to the neat GelMA resins.

The GelMA/PCL-MA hybrid resin was developed for use in scaffolds suitable for growing *in vitro* intestinal tissue. Caco-2 cell monolayers, which are traditionally used as *in vitro* intestinal tissue models, are typically cultured on a porous membrane attached to a cell culture insert to ensure unrestricted penetration of oxygen and nutrients through the substrate to the basolateral side of the epithelial cells. In the hybrid material, highly swelling GelMA enabled the albumin penetration through the material, while non-swelling neat PCL-MA samples were virtually non-permeable. The increased swelling and thereby albumin permeability of the GelMA/PCL-MA hybrid samples compared to neat PCL-MA samples could be attributed to the high amount of polar groups in GelMA and thereby its hydrophilicity and also the lower crosslinking density of the hybrid samples compared to the PCL-MA that allowed less restricted penetration of water and albumin through the material.

In prior art, low concentration (7 %) GelMA hydrogels have been studied and found to be highly permeable to 70 kDa dextran molecules. Besides the increased albumin permeability, the GelMA/PCL-MA hybrid samples resulted in significantly lower elastic modulus (10-16 kPa) compared to neat PCL-MA samples (206 kPa). The elastic modulus of the GelMA gels crosslinked in formamide were consistent with the GelMA gels crosslinked in aqueous solution elsewhere.

In prior art, a higher compressive modulus of PGelMA compared to FGelMA was observed and attributed either to its higher degree of methacrylation or a higher number of hydrophobic interactions and imino acids providing structural stability. Within the framework of the present invention, there was no difference between the fish and porcine GelMA-containing samples. Both FGelMA and PGelMA had a high methacrylation degree, while the PGelMA resin contained more formamide to ensure the suitable viscosity for SLA. Therefore, the lower polymer concentration of PGelMA samples assumedly canceled out the possible strengthening effect of its more hydrophobic amino acid composition. Interestingly, the addition of PCL-MA into GelMA resin did not increase the elastic modulus compared to neat GelMA samples, which may indicate a slight phase separation of the materials even though macroscopically this was not visible in the resins. Besides being softer than neat PCL-MA samples, the hybrid samples degraded faster in the presence of collagenase due to the enzymatically labile peptide bonds in GelMA. While PCL-MA did not virtually lose mass within 96 hours, the GelMA/PCL-MA hybrid materials lost 89-75 % of their mass. FGelMA-containing samples decreased clearly faster than PGelMA-containing samples, which was consistent with the previous literature, where the faster degradation of FGelMA hydrogels was attributed to the lower amount of imino acids in FGelMA providing less structural integrity than in PGelMA.

To test the use of GelMA/PCL-MA scaffolds for supporting growth of intestinal cells, Caco-2 cells were cultured on the 3D printed samples. When cultured on an optimal substrate, these cells differentiate into polarized, micro villi-containing epithelial cells. Addition of GelMA into the PCL-MA resin resulted in clearly improved Caco-2 cell adhesion compared to the neat PCL-MA substrates, which could be attributed to the abundant RGD cell adhesive sequences in GelMA. The PGelMA-containing samples supported enhanced Caco-2 adhesion and proliferation compared to the FGelMA-containing samples. In addition to the proliferation study, the early differentiation of Caco-2 cells on the 3D printed hybrid materials was evaluated by following the formation of tight junctions and micro villi structures. Even though both of the GelMA/PCL-MA hybrid materials promoted tight junction formation between Caco-2 cells, SEM images revealed the more dense early formation of microvilli structure on the PGelMA/PCL-MA samples compared to the FGelMA/PCL-MA samples.

To study the use of more complex and biological dECM-MA prepolymer instead of GelMA in SLA printing, a novel dECM-MA/PCL-MA hybrid resin was formulated. The dECM-MA was first obtained by solubilizing a decellularized rat liver with pepsin digestion and subsequently methacrylating the dECM with the same pH-controlled protocol as gelatin. The TNBSA test showed nearly full methacrylation of the primary amines, and the $^1$H NMR analysis revealed the chemical structure of dECM-MA to be similar with GelMA, which was attributed to the methacrylation of collagen that is abundantly present in a rat liver among laminin, fibronectin, glycosaminoglycans, and growth factors. dECM is an attractive material for use in 3D printing of tissue scaffolds as it recapitulate the complexity of native tissue better than GelMA and can provide a natural, tissue-specific environment for encapsulated or seeded cells. While non-modified pepsin-digested dECM has been used in SLA to form one-layer cellular structures blended with photocrosslinkable GelMA, photocrosslinkable dECM for the multi-layer SLA printing blended with PCL-MA was first developed in the framework of the present invention. Dissolving PCL-MA in benzyl alcohol before mixing with dECM-MA in formamide led to a homogenous dECM-MA/PCL-MA resin.

### Conclusions

Due to its difficult viscosity control, GelMA has not been widely used in SLA-based 3D printing of tissue scaffolds. By using formamide as a solvent for the resin formulations, a photocrosslinkable GelMA composition was obtained. By adding PCL-MA, a GelMA/PCL-MA hybrid resin was obtained that allowed high-resolution 3D printing without premature physical gelation of the material. The resulting hybrid material captured the cell adhesiveness of GelMA and consequently supported proliferation and differentiation of Caco-2 cell into micro-villi containing epithelial cells. It is possible to replace GelMA by functionalized, in particular methacryl-functionalized, dECM in the pure resin or the hybrid resin for SLA 3D printing. The new resins significantly expand and diversify the use of GelMA and dECM in SLA 3D printing. This will particularly promote the 3D printing of tissue scaffolds that simultaneously require an optimized fidelity, intrinsic permeability, and enhanced cell adhesiveness.

### List of references concerning relevant technical background

1. Mechels, F., Feijen, J. and Grijpma, D. Biomaterials 31, 2010.

2. Mota, C., Puppi, D., Chiellini, F. and Chiellini, E. J Tissue Eng Regen Med 9, 2015.

3. Murphy, S. V and Atala, A. Nat Biotechnol 32, 2014.

4. Green, B.J., Worthington, K.S., Thompson, J.R., Bunn, S.J., Rethwisch, M., Kaalberg, E.E., Jiao, C., Wiley, L.A., Mullins, R.F., Stone, E.M., Sohn, E.H., Tucker, B.A. and Guymon, C.A. Biomacromolecules 19, 2018.

5. Guess, T.R., Chambers, R.S. and Hinnerichs, T.D. Epoxy and acrylate sterolithography resins: in-situ property measurements. Technical report from U.S. Department of Energy Office of Scientific and Technical Information, 1996.

6. Cui, J., Yu, G. and Pan, C. J Appl Polym Sci 131, 2014.

7. Elomaa, L., Teixeira, S., Hakala, R., Korhonen, H., Grijpma, D.W. and Seppälä, J.V. Acta Biomater 7, 2011.

8. Djabourov, M., Leblond, J. and Papon, P. J Phys 49, 1988.

9. Pepelanova, I., Kruppa, K., Scheper, T. and Lavrentieva, A. Bioengineering 5, 2018.

10. Kaemmerer, E., Melchels, F.P.W., Holzapfel, B.M., Meckel, T., Hutmacher, D.W. and Loessner, D. Acta Biomater 10, 2014.

11. Wang, Z., Kumar, H., Tian, Z., Jin, X., Holzman, J.F., Menard, F. and Kim, K. ACS Appl Mater Interfaces 10, 2018.

12. Ma, X., Yu, C., Wang, P., Xu, W., Wan, X., Sun Edwin Lai, C., Liu, J., Koroleva-Maharajh, A. and Chen, S. Rapid 3D Bioprinting of Decellularized Extracellular Matrix with Regionally Varied Mechanical Properties and Biomimetic Microarchitecture. Biomaterials 185, 2018.

13. Lee, B.H., Shirahama, H., Cho, N.-J. and Tan, L.P. Efficient and controllable synthesis of highly substituted gelatin methacrylamide for mechanically stiff hydrogels. RSC Adv 5, 2015.

14. Struecker, B., Butter, A., Hillebrandt, K., Polenz, D., Reutzel-Selke, A., Tang, P., Lippert, S., Leder, A., Rohn, S., Geisel, D., Denecke, T., Aliyev, K., Jöhrens, K., Raschzok, N., Neuhaus, P., Pratschke, J. and Sauer, I.M. Improved rat liver decellularization by arterial perfusion under oscillating pressure conditions. J Tissue Eng Regen Med 11, 2017.

15. Rothrauff, B.B., Coluccino, L., Gottardi, R., Ceseracciu, L., Scaglione, S., Goldoni, L. and Tuan, R.S. Efficacy of thermoresponsive, photocrosslinkable hydrogels derived from decellularized tendon and cartilage extracellular matrix for cartilage tissue engineering. J Tissue Eng Regen Med 12, 2018.

16. US 2014/0167300 A1

17. US 9,453,142 B2

18. US 2017/0368225 A1

19. US 2018/0312705 A1

**Claims**

1. Photocurable composition for 3D printing, comprising

   - at least one photocurable prepolymer chosen from the group consisting of functionalized gelatin bearing a first functional group and functionalized decellularized extracellular matrix bearing a second functional group,
   - at least one solvent, the solvent being formamide, and
   - at least one photoinitiator.

2. Photocurable composition according to claim 1, **characterized in that** the composition further comprises functionalized poly($\epsilon$-caprolactone) bearing a third functional group.

3. Photocurable composition according to claim 1 or 2, **characterized in that** the first functional group, the second functional group, and - insofar as referring back to claim 2 - the third functional group are independently chosen from each other from the group consisting of methacryloyl, methacrylate, methacrylamide, acryloyl, thiol, vinyl, vinylene, allyl, alkynyl, epoxide, hydroxyl, and conjugated dienyl groups.

4. Photocurable composition according to claim 3, **characterized in that** the first functional group, the second functional group, and - insofar as referring back to claim 2 - the third functional group can only be a thiol group if any compound of the composition comprises a functional group chosen from the group consisting of methacryloyl, methacrylate, methacrylamide, acryloyl, vinyl, vinylene, allyl, alkynyl, epoxide, hydroxyl and conjugated dienyl groups.

5. Photocurable composition according to any of the preceding claims, **characterized in that** the photocurable prepolymer is or comprises gelatin methacryloyl.

6. Photocurable composition according to claim 5, **characterized in that** the gelatin methacryloyl is fish gelatin methacryloyl or porcine gelatin methacryloyl.

7. Photocurable composition according to any of the preceding claims, **characterized in that** the composition further comprises a light-absorbing colorant.

8. Photocurable composition according to any of the preceding claims, **characterized in that** the composition further comprises a crosslinker.

9. Photocurable composition according to claim 8, **characterized in that** the crosslinker is a low molecular weight molecule comprising at least two reactive groups chosen independently from each other from the group consisting of methacryloyl, methacrylate, methacrylamide, acryloyl, thiol, vinyl, vinylene, allyl, alkynyl, epoxide, hydroxyl, and conjugated dienyl groups.

10. Photocurable composition according to any of the preceding claims, **characterized in that** the composition comprises 30 to 95 percent by weight, based on the weight of the photocurable prepolymer, of the solvent, 0.5 to 5 percent by weight, based on the weight of the photocurable prepolymer, of the photoinitiator, 0 to 100 percent by weight, based on the weight of the photocurable prepolymer, of functionalized poly($\varepsilon$-caprolactone) bearing a third functional group, 0 to 0.5 percent by weight, based on the weight of the photocurable prepolymer, of a light-absorbing colorant, 0 to 20 percent by weight, based on the weight of the photocurable prepolymer, of a crosslinker.

11. Use of a photocurable composition according to any of the preceding claims as resin for manufacturing a three-dimensional object by 3D printing.

12. Use according to claim 11, **characterized in that** the three-dimensional object serves as scaffold for growing cells or tissue on it.

13. Method for manufacturing a three-dimensional object from a resin by 3D printing the resin, wherein the method comprises the following steps:

a) providing the resin onto a carrier or a layer already formed from the resin,
b) illuminating those sections of the resin in a defined layer that are intended to crosslink,
c) letting automatically crosslink the illuminated sections of the resin, and
d) repeating steps a) to c) for a desired amount of layers,

**characterized**
**in that** the resin comprises i) at least one photocurable prepolymer chosen from the group consisting of functionalized gelatin bearing a first functional group and functionalized decellularized extracellular matrix bearing a second functional group, ii) at least one solvent, the solvent being formamide, and iii) at least one photoinitiator.

14. Method according to claim 13, **characterized in that** the method is carried out in a temperature range of 15 °C to 37 °C.

**Patentansprüche**

1. Lichthärtbare Zusammensetzung für den 3D-Druck, umfassend

- mindestens ein lichthärtbares Vorpolymer, das aus der Gruppe ausgewählt ist, die aus funktionalisierter Gelatine mit einer ersten funktionellen Gruppe und funktionalisierter dezellularisierter extrazellulärer Matrix mit einer zweiten funktionellen Gruppe besteht,
- mindestens ein Lösungsmittel, wobei das Lösungsmittel Formamid ist, und
- mindestens einen Photoinitiator.

2. Lichthärtbare Zusammensetzung nach Anspruch 1, **dadurch gekennzeichnet, dass** die Zusammensetzung ferner funktionalisiertes Poly($\epsilon$-caprolacton) mit einer dritten funktionellen Gruppe umfasst.

3. Lichthärtbare Zusammensetzung nach Anspruch 1 oder 2, **dadurch gekennzeichnet, dass** die erste funktionelle Gruppe, die zweite funktionelle Gruppe und - soweit auf Anspruch 2 Bezug genommen wird - die dritte funktionelle Gruppe unabhängig voneinander aus der aus Methacryloyl-, Methacrylat-, Methacrylamid-, Acryloyl-, Thiol-, Vinyl-, Vinylen-, Allyl-, Alkinyl-, Epoxid-, Hydroxyl- und konjugierten Dienylgruppen bestehenden Gruppe ausgewählt sind.

4. Lichthärtbare Zusammensetzung nach Anspruch 3, **dadurch gekennzeichnet, dass** die erste funktionelle Gruppe, die zweite funktionelle Gruppe und - soweit auf Anspruch 2 Bezug genommen wird - die dritte funktionelle Gruppe nur dann eine Thiolgruppe sein kann, wenn irgendeine Verbindung der Zusammensetzung eine funktionelle Gruppe umfasst, die aus der aus Methacryloyl-, Methacrylat-, Methacrylamid-, Acryloyl-, Vinyl-, Vinylen-, Allyl-, Alkinyl-, Epoxid-, Hydroxyl- und konjugierten Dienylgruppen bestehenden Gruppe ausgewählt ist.

5. Lichthärtbare Zusammensetzung nach einem der vorhergehenden Ansprüche, **dadurch gekennzeichnet, dass** das lichthärtbare Vorpolymer Gelatinemethacryloyl ist oder umfasst.

6. Lichthärtbare Zusammensetzung nach Anspruch 5, **dadurch gekennzeichnet, dass** es sich bei dem Gelatinemethacryloyl um Fischgelatine-Methacryloyl oder Schweinegelatine-Methacryloyl handelt.

7. Lichthärtbare Zusammensetzung nach einem der vorhergehenden Ansprüche, **dadurch gekennzeichnet, dass** die Zusammensetzung ferner ein lichtabsorbierendes Farbmittel umfasst.

8. Lichthärtbare Zusammensetzung nach einem der vorhergehenden Ansprüche, **dadurch gekennzeichnet, dass** die Zusammensetzung ferner einen Vernetzer umfasst.

9. Lichthärtbare Zusammensetzung nach Anspruch 8, **dadurch gekennzeichnet, dass** der Vernetzer ein Molekül mit niedrigem Molekulargewicht ist, das mindestens zwei reaktive Gruppen umfasst, die unabhängig voneinander aus der aus Methacryloyl-, Methacrylat-, Methacrylamid-, Acryloyl-, Thiol-, Vinyl-, Vinylen-, Allyl-, Alkinyl-, Epoxid-, Hydroxyl- und konjugierten Dienylgruppen bestehenden Gruppe ausgewählt sind.

10. Lichthärtbare Zusammensetzung nach einem der vorhergehenden Ansprüche, **dadurch gekennzeichnet, dass** die Zusammensetzung 30 bis 95 Gew.-%, bezogen auf das Gewicht des lichthärtbaren Vorpolymers, des Lösungsmittels, 0,5 bis 5 Gew.-%, bezogen auf das Gewicht des lichthärtbaren Vorpolymers, des Photoinitiators, 0 bis 100 Gew.-%, bezogen auf das Gewicht des lichthärtbaren Vorpolymers, funktionalisiertes Poly($\epsilon$-caprolacton) mit einer dritten funktionellen Gruppe, 0 bis 0,5 Gew.-%, bezogen auf das Gewicht des lichthärtbaren Vorpolymers, eines lichtabsorbierenden Farbmittels, 0 bis 20 Gew.-%, bezogen auf das Gewicht des lichthärtbaren Vorpolymers, eines Vernetzers umfasst.

11. Verwendung einer lichthärtbaren Zusammensetzung nach einem der vorhergehenden Ansprüche als Harz zur Herstellung eines dreidimensionalen Objekts durch 3D-Druck.

12. Verwendung nach Anspruch 11, **dadurch gekennzeichnet, dass** das dreidimensionale Objekt als Gerüst zum Züchten von Zellen oder Gewebe darauf dient.

13. Verfahren zur Herstellung eines dreidimensionalen Objekts aus einem Harz durch 3D-Drucken des Harzes, wobei das Verfahren die folgenden Schritte umfasst:

a) Aufbringen des Harzes auf einen Träger oder eine bereits aus dem Harz gebildete Schicht,
b) Beleuchten derjenigen Abschnitte des Harzes in einer definierten Schicht, die vernetzt werden sollen,
c) die beleuchteten Abschnitte des Harzes automatisch vernetzen lassen, und
d) Wiederholen der Schritte a) bis c) für eine gewünschte Menge an Schichten,

**dadurch gekennzeichnet,**
**dass** das Harz i) mindestens ein lichthärtbares Vorpolymer, das aus der aus funktionalisierter Gelatine mit einer ersten funktionellen Gruppe und funktionalisierter dezellularisierter extrazellulärer Matrix mit einer zweiten funktionellen Gruppe bestehenden Gruppe ausgewählt ist, ii) mindestens ein Lösungsmittel, wobei das Lösungsmittel Formamid ist, und iii) mindestens einen Photoinitiator umfasst.

14. Verfahren nach Anspruch 13, **dadurch gekennzeichnet, dass** das Verfahren in einem Temperaturbereich von 15 °C bis 37 °C durchgeführt wird.

**Revendications**

1. Composition photodurcissable pour l'impression 3D, comprenant

    - au moins un prépolymère photodurcissable choisi dans le groupe constitué par la gélatine fonctionnalisée portant un premier groupe fonctionnel et la matrice extracellulaire décellularisée fonctionnalisée portant un deuxième groupe fonctionnel,
    - au moins un solvant, le solvant étant le formamide, et
    - au moins un photo-initiateur.

2. Composition photodurcissable selon la revendication 1, **caractérisée en ce que** la composition comprend en outre du poly($\varepsilon$-caprolactone) fonctionnalisé portant un troisième groupe fonctionnel.

3. Composition photodurcissable selon la revendication 1 ou 2, **caractérisée en ce que** le premier groupe fonctionnel, le deuxième groupe fonctionnel et - dans la mesure où l'on se réfère à la revendication 2 - le troisième groupe fonctionnel sont choisis indépendamment les uns des autres dans le groupe constitué par les groupes méthacryloyle, méthacrylate, méthacrylamide, acryloyle, thiol, vinyle, vinylène, allyle, alcynyle, époxyde, hydroxyle et diényle conjugué.

4. Composition photodurcissable selon la revendication 3, **caractérisée en ce que** le premier groupe fonctionnel, le deuxième groupe fonctionnel et - dans la mesure où l'on se réfère à la revendication 2 - le troisième groupe fonctionnel ne peuvent être un groupe thiol que si un composé quelconque de la composition comprend un groupe fonctionnel choisi dans le groupe constitué par les groupes méthacryloyle, méthacrylate, méthacrylamide, acryloyle, vinyle, vinylène, allyle, alcynyle, époxyde, hydroxyle et diényle conjugué.

5. Composition photodurcissable selon l'une quelconque des revendications précédentes, **caractérisée en ce que** le prépolymère photodurcissable est ou comprend de la gélatine méthacryloyle.

6. Composition photodurcissable selon la revendication 5, **caractérisée en ce que** la gélatine méthacryloyle est une gélatine méthacryloyle de poisson ou une gélatine méthacryloyle de porc.

7. Composition photodurcissable selon l'une quelconque des revendications précédentes, **caractérisée en ce que** la composition comprend en outre un colorant absorbant la lumière.

8. Composition photodurcissable selon l'une quelconque des revendications précédentes, **caractérisée en ce que** la composition comprend en outre un agent de réticulation.

9. Composition photodurcissable selon la revendication 8, **caractérisée en ce que** l'agent de réticulation est une molécule de faible poids moléculaire comprenant au moins deux groupes réactifs choisis indépendamment les uns des autres dans le groupe constitué par les groupes méthacryloyle, méthacrylate, méthacrylamide, acryloyle, thiol, vinyle, vinylène, allyle, alcynyle, époxyde, hydroxyle et diényle conjugué.

10. Composition photodurcissable selon l'une quelconque des revendications précédentes, **caractérisée en ce que** la composition comprend 30 à 95 pour cent en poids, par rapport au poids du prépolymère photodurcissable, du solvant, 0,5 à 5 pour cent en poids, par rapport au poids du prépolymère photodurcissable, du photo-initiateur, 0 à 100 pour cent en poids, par rapport au poids du prépolymère photodurcissable, de poly($\varepsilon$-caprolactone) fonctionnalisé portant un troisième groupe fonctionnel, 0 à 0,5 pour cent en poids, par rapport au poids du prépolymère photodurcissable, d'un colorant absorbant la lumière, 0 à 20 pour cent en poids, par rapport au poids du prépolymère photodurcissable, d'un agent de réticulation.

11. Utilisation d'une composition photodurcissable selon l'une quelconque des revendications précédentes comme résine pour la fabrication d'un objet tridimensionnel par impression 3D.

12. Utilisation selon la revendication 11, **caractérisée en ce que** l'objet tridimensionnel sert d'échafaudage pour la croissance de cellules ou de tissus sur celui-ci.

13. Procédé de fabrication d'un objet tridimensionnel à partir d'une résine par impression 3D de la résine, le procédé comprenant les étapes suivantes :

a) prévoir la résine sur un support ou une couche déjà formée à partir de la résine,
b) éclairer les sections de la résine dans une couche définie qui sont destinées à réticuler,
c) laisser réticuler automatiquement les sections éclairées de la résine, et
d) répéter les étapes a) à c) pour une quantité désirée de couches,

**caractérisé**
**en ce que** la résine comprend i) au moins un prépolymère photodurcissable choisi dans le groupe constitué par la gélatine fonctionnalisée portant un premier groupe fonctionnel et la matrice extracellulaire décellularisée fonctionnalisée portant un deuxième groupe fonctionnel, ii) au moins un solvant, le solvant étant le formamide, et iii) au moins un photo-initiateur.

14. Procédé selon la revendication 13, **caractérisé en ce que** le procédé est réalisé dans une plage de température de 15 °C à 37 °C.

GelMA or ECM-MA

PCL-MA =

Photoinitiator

## FIG 1A

CAD model

Layers for 3D prining

DLP SLA

Movable platfrom

Resin

3D structure

Light source

Digital micro-mirror device

## FIG 1B

FIG 2A

FIG 2B

FIG 3A

FIG 3B

FIG 4A

FIG 4B

FIG 5A

FIG 5B

FIG 5C

FIG 5D

FIG 5E

FIG 6A

FIG 6B

FIG 7

FIG 8

FIG 9A

FIG 9B

FIG 9C

FIG 9D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2019119429 A
- US 20140167300 A1
- US 9453142 B2
- US 20170368225 A1
- US 20180312705 A1

### Non-patent literature cited in the description

- **MECHELS, F. ; FEIJEN, J. ; GRIJPMA, D.** *Biomaterials,* 2010, vol. 31
- **MOTA, C. ; PUPPI, D. ; CHIELLINI, F. ; CHIELLINI, E.** *J Tissue Eng Regen Med,* 2015, vol. 9
- **MURPHY, S. V ; ATALA, A.** *Nat Biotechnol,* 2014, vol. 32
- **GREEN, B.J. ; WORTHINGTON, K.S. ; THOMPSON, J.R. ; BUNN, S.J. ; RETHWISCH, M. ; KAALBERG, E.E. ; JIAO, C. ; WILEY, L.A. ; MULLINS, R.F. ; STONE, E.M.** *Biomacromolecules,* 2018, vol. 19
- **GUESS, T.R. ; CHAMBERS, R.S. ; HINNERICHS, T.D.** Epoxy and acrylate sterolithography resins: in-situ property measurements. *Technical report from U.S. Department of Energy Office of Scientific and Technical Information,* 1996
- **CUI, J. ; YU, G. ; PAN, C.** *J Appl Polym Sci,* 2014, vol. 131
- **ELOMAA, L. ; TEIXEIRA, S. ; HAKALA, R. ; KORHONEN, H. ; GRIJPMA, D.W. ; SEPPÄLÄ, J.V.** *Acta Biomater,* 2011, vol. 7
- **DJABOUROV, M. ; LEBLOND, J. ; PAPON, P.** *J Phys,* 1988, vol. 49
- **PEPELANOVA, I. ; KRUPPA, K. ; SCHEPER, T. ; LAVRENTIEVA, A.** *Bioengineering,* 2018, vol. 5
- **KAEMMERER, E. ; MELCHELS, F.P.W. ; HOLZAPFEL, B.M. ; MECKEL, T. ; HUTMACHER, D.W. ; LOESSNER, D.** *Acta Biomater,* 2014, vol. 10
- **WANG, Z. ; KUMAR, H. ; TIAN, Z. ; JIN, X. ; HOLZMAN, J.F. ; MENARD, F. ; KIM, K.** *ACS Appl Mater Interfaces,* 2018, vol. 10
- **MA, X. ; YU, C. ; WANG, P. ; XU, W. ; WAN, X. ; SUN EDWIN LAI, C. ; LIU, J. ; KOROLEVA-MAHARAJH, A. ; CHEN, S.** Rapid 3D Bioprinting of Decellularized Extracellular Matrix with Regionally Varied Mechanical Properties and Biomimetic Microarchitecture. *Biomaterials,* 2018, vol. 185
- **LEE, B.H. ; SHIRAHAMA, H. ; CHO, N.-J. ; TAN, L.P.** Efficient and controllable synthesis of highly substituted gelatin methacrylamide for mechanically stiff hydrogels. *RSC Adv,* 2015, vol. 5
- **STRUECKER, B. ; BUTTER, A. ; HILLEBRANDT, K. ; POLENZ, D. ; REUTZEL-SELKE, A. ; TANG, P. ; LIPPERT, S. ; LEDER, A. ; ROHN, S. ; GEISEL, D.** Improved rat liver decellularization by arterial perfusion under oscillating pressure conditions. *J Tissue Eng Regen Med,* 2017, vol. 11
- **ROTHRAUFF, B.B. ; COLUCCINO, L. ; GOTTARDI, R. ; CESERACCIU, L. ; SCAGLIONE, S. ; GOLDONI, L. ; TUAN, R.S.** Efficacy of thermoresponsive, photocrosslinkable hydrogels derived from decellularized tendon and cartilage extracellular matrix for cartilage tissue engineering. *J Tissue Eng Regen Med,* 2018, vol. 12